# EUROPÄISCHE PATENTSCHRIFT

(11) **EP 2 734 203 B1**
(45) Veröffentlichungstag und Bekanntmachung des Hinweises auf die Patenterteilung: **06.07.2016**
(21) Anmeldenummer: 12735554.3
(22) Anmeldetag: 17.07.2012
(51) Int. Cl.: A61K 47/02, A61K 31/43, A61K 31/7036, A61K 38/12, A61P 11/00, A61P 31/00, A61P 31/04, A61K 9/00

(54) **PHARMAZEUTISCHE ZUSAMMENSETZUNG ZUR BEHANDLUNG EINER ATEMWEGSERKRANKUNG**
PHARMACEUTICAL COMPOSITION FOR THE TREATMENT OF RESPIRATORY DISORDERS
COMPOSITION PHARMACEUTIQUE DESTINÉE AU TRAITEMENT DE TROUBLES RESPIRATOIRES

(30) Priorität: 21.07.2011 DE 102011108227
(43) Veröffentlichungstag der Anmeldung: 28.05.2014
(73) Patentinhaber: Eberhard-Karls-Universität Tübingen Universitätsklinikum, 72076 Tübingen (DE)
(72) Erfinder: DOERING, Gerd, 72074 Tuebingen (DE); RIETHMUELLER, Joachim, 72076 Tuebingen (DE); HERRMANN, Gloria, 72076 Tuebingen (DE)
(74) Vertreter: Witte, Weller & Partner Patentanwälte mbB
(86) Internationale Anmeldenummer: PCT/EP2012/063995
(87) Internationale Veröffentlichungsnummer: WO 2013/011019

(56) Entgegenhaltungen:
- GHANNAM D E ET AL: "Inhaled aminoglycosides in cancer patients with ventilator-associated Gram-negative bacterial pneumonia: safety and feasibility in the era of escalating drug resistance", EUROPEAN JOURNAL OF CLINICAL MICROBIOLOGY & INFECTIOUS DISEASES, SPRINGER, BERLIN, DE, Bd. 28, Nr. 3, 28. August 2008 (2008-08-28), Seiten 253-259, XP019663291, ISSN: 1435-4373
- PINTADO ET AL: "Intravenous colistin sulphomethate sodium for therapy of infections due to multidrug-resistant gram-negative bacteria", JOURNAL OF INFECTION, ACADEMIC PRESS, LONDON, GB, Bd. 56, Nr. 3, 15. Februar 2008 (2008-02-15), Seiten 185-190, XP022491581, ISSN: 0163-4453, DOI: 10.1016/J.JINF.2008.01.003
- AKINDIPE ET AL: "Left Pneumonectomy in a Patient With a Chronically Infected Allograft", JOURNAL OF HEART AND LUNG TRANSPLANTATION, MOSBY-YEAR BOOK, INC., ST LOUIS, MO, US, Bd. 26, Nr. 10, 3. Oktober 2007 (2007-10-03), Seiten 1072-1074, XP022283356, ISSN: 1053-2498, DOI: 10.1016/J.HEALUN.2007.07.015
- GLORIA HERRMANN ET AL: "Colistin-Tobramycin Combinations Are Superior to Monotherapy Concerning the Killing of Biofilm Pseudomonas aeruginosa", THE JOURNAL OF INFECTIOUS DISEASES, Bd. 202, Nr. 10, 15. November 2010 (2010-11-15), Seiten 1585-1592, XP55040392, ISSN: 0022-1899, DOI: 10.1086/656788
- MACGOWAN A P ET AL: "In vitro assessment of colistin's antipseudomonal antimicrobial interactions with other antibiotics", CLINICAL MICROBIOLOGY AND INFECTION, WILEY-BLACKWELL PUBLISHING LTD, UNITED KINGDOM, SWITZERLAND, Bd. 5, Nr. 1, 1. Januar 1999 (1999-01-01), Seiten 32-36, XP009163523, ISSN: 1198-743X [gefunden am 2008-10-27]
- D. HILL ET AL: "Antibiotic Susceptibilities of Pseudomonas aeruginosa Isolates Derived from Patients with Cystic Fibrosis under Aerobic, Anaerobic, and Biofilm Conditions", JOURNAL OF CLINICAL MICROBIOLOGY, Bd. 43, Nr. 10, 1. Oktober 2005 (2005-10-01), Seiten 5085-5090, XP55040747, ISSN: 0095-1137, DOI: 10.1128/JCM.43.10.5085-5090.2005
- K. E. N. MILNE ET AL: "Combination testing of multidrug-resistant cystic fibrosis isolates of Pseudomonas aeruginosa: use of a new parameter, the susceptible breakpoint index", JOURNAL OF ANTIMICROBIAL CHEMOTHERAPY, Bd. 65, Nr. 1, 27. Oktober 2009 (2009-10-27), Seiten 82-90, XP55040748, ISSN: 0305-7453, DOI: 10.1093/jac/dkp384
- KUMAR A ET AL: "IN-VITRO ACTIVITY OF MULTIPLE ANTIMICROBIAL COMBINATIONS AGAINST PSEUDOMONAS-CEPACIA ISOLATES", CHEMOTHERAPY, S. KARGER AG, CH, Bd. 35, Nr. 4, 1. Januar 1989 (1989-01-01) , Seiten 246-253, XP009163518, ISSN: 0009-3157
- DÖRING, GERD, HOIBY, NIELS FOR THE CONSENSUS STUDY GROUP: "Early intervention and prevention of lung disease in cystic fibrosis: a European consensus.", J. CYST. FIBROS., Bd. 3, Nr. 2, 1. Juni 2004 (2004-06-01), Seiten 67-91, XP002685078,

## Beschreibung

Die vorliegende Erfindung betrifft eine pharmazeutische Zusammensetzung sowie die Verwendung derselben zur Behandlung einer Atemwegserkrankung.

Die Entwicklung von wirksamen Behandlungskonzepten bei Atemwegsinfektionen stellt eine der großen Herausforderungen in unserer Zeit dar. Dies gilt insbesondere für chronisch-bakterielle Lungenerkrankungen, wie sie bei Patienten mit chronisch obstruktiver Lungenerkrankung (engl. "chronic obstructive pulmonary disease", COPD) oder cystischer Fibrose (engl. "cystic fibrosis", CF) auftreten.

COPD ist eine chronische progressive Störung des Lungenparenchyms, von der etwa 9 % der Bevölkerung betroffen sind. Global gesehen ist COPD die vierthäufigste Todesursache, die im Jahre 2003 für ungefähr 2,7 Millionen Todesfälle verantwortlich war. Die progrediente Lungenschädigung erleichtert bakterielle Lungeninfektionen, die die Lungenfunktionsstörung negativ beeinflussen. Etwa 10 % der Patienten mit fortgeschrittener COPD leiden unter einer chronischen Lungeninfektion mit *Pseudomonas aeruginosa.* Andere opportunistisch-pathogene Bakterien umfassen nicht typisierbare H. *influenzae* (NTHI), *Moraxella catarrhalis, Streptococcus pneumoniae* und Anaerobier, die in den Luftwegen von etwa 20 bis 25 % der klinisch stabilen COPD-Patienten nachweisbar sind.

Auch bei der Erbkrankheit CF führen sekundär erworbene chronisch-bakterielle Lungeninfektionen zu einer verminderten Lebenserwartung. Weltweit gibt es schätzungsweise 100.000 CF-Patienten, von denen der überwiegende Anteil an chronischen Lungeninfektionen leidet. Neuere Untersuchungen haben gezeigt, dass in den Luftwegen der CF-Patienten viele verschiedene Bakterienspezies vorhanden sind, darunter *Pseudomonas aeruginosa, Staphylococcus aureus,* NTHI, komplexe Stämme von *Burkholderia cepacia, Acinetobacter baumanii* sowie viele weitere Spezies strikt anaerober Bakterien wie *Prevotella intermedia.*

Sowohl bei COPD- als auch bei CF-Patienten führen persistierende bakterielle Lungeninfektionen zum Einstrom neutrophiler Granulozyten aus dem Blut in die betroffenen Atemwege, wodurch visköse Mukusplaques gebildet werden. Dadurch wird die Selbstreinigung der Lunge über Zilientätigkeit vermindert und die Wirksamkeit von Antibiotika herabgesetzt. Die verminderten Antibiotika-Aktivitäten erleichtern zudem die Resistenzbildung in den Bakterien, insbesondere wenn nur ein einziges Antibiotikum verabreicht wird. Dies trägt wiederum zu ihrer Persistenz bei und erhöht die Morbidität wie die Mortalität bei den Patienten.

Bei CF-Patienten werden vorwiegend Lungeninfektionen durch *Pseudomonas aeroginosa* mit verschiedenen Antibiotika behandelt. Bei COPD-Patienten gibt es keine rationalen Therapiestrategien in diesem Kontext.

Der Nachteil der derzeitigen Behandlungsstrategien für CF-Patienten besteht darin, dass die Vielzahl anderer bakterieller Pathogene, die neben *Pseudomonas aeruginosa* in den Atemwegen der Patienten zur Lungenerkrankung beitragen, unberücksichtigt bleiben.

Auch die zur Behandlung von COPD-Patienten derzeit eingesetzten antibiotikabasierenden Therapien haben sich bislang nicht bewährt.

Ghannam et al. (2009), Inhaled Aminoglycosides in Cancer Patients With Ventilator-Associated Gram-Negative Bacterial Pneumonia: Safety and Feasibility in the Era of Escalating Drug Resistance, Eur. J. Microbial. Infect. Dis. 28:253-259, beschreiben eine retrospektive Studie, in der die Verabreichung von Aminoglykosiden und Colistin bei Krebspatienten mit ventilatorassoziierter bakterieller Gram-negativer Pneumonie untersucht wurde. Dabei erhielt ein Patient die kombinierte Verabreichung von Colistin und Tobramycin per Inhalation und außerdem systemisch Imipenem. Pintado et al. (2008), Intravenous Colistin Sulphomethate Sodium for Therapy of Infections Due to Multidrug-Resistant Gram-Negative Bacteria, Journal of Infection 56: 185-190, beschreiben eine retrospektive Studie, in der die intravenöse Verabreichung von Colistin zur Therapie von Infektionen durch multiresistente Gram-negative Bakterien untersucht wird. Erwähnt ist, dass 15 Patienten außerdem in Kombination Beta-Lactame und Aminoglykoside erhielten. Als Beispiel für Beta-Lactame werden u.a. Imipenem und Meropenem genannt. Als Beispiel für Aminoglykoside werden Tobramycin und Amikacin genannt.
Akindipe et al. (2007), Left Pneumonectomy in a Patient With a Chronically Infected Allograft, beschreiben eine Therapie an einer schwer lungenerkrankten Person, in der intravenös Meropenem und Tobramycin sowie per Inhalation Colistin verabreicht wurde.

Vor diesem Hintergrund ist es die der Erfindung zugrundeliegende Aufgabe, eine pharmazeutische Zusammensetzung bereitzustellen, mit der die vorstehend genannten Nachteile aus dem Stand der Technik vermindert, vorzugsweise vermieden werden.

Diese Aufgabe wird durch eine pharmazeutische Zusammensetzung gelöst, die eine Kombination von zumindest einem Carbapenem, Polymyxin und Aminoglykosid sowie einen pharmazeutisch akzeptablen Träger aufweist, die für eine Applikation im Wege der Inhalation ausgestaltet ist.

Carbapeneme, ursprünglich als Thienamycine bezeichnet, sind sog. β-Lactam-Antibiotika mit breitem Wirkungsspektrum. Wichtige Vertreter sind Imipenem, Ertapenem, Meropenem und Doripenem. Carbapeneme hemmen die Zellwandsynthese der Bakterien durch Bindung an Penicillinbindeproteine.

Polymyxine bilden eine Gruppe von Polypeptid-Antibiotika, die chemisch verzweigte, zyklische Dekapeptide darstellen. Ähnlich wie die Zellmembran besitzen sie eine Polarität, die durch die in ihnen enthaltenden Aminosäuren und endständige hydrophobe Fettsäuren entsteht. Dadurch werden sie in die Zellmembran gram-negativer Bakterien eingelagert und stören deren Permeabilität. Aufgrund dessen besitzen diese Antibiotika eine bakterizide Wirkung. Zu den Polymyxinen zählen bspw. Polymyxin B, Polymyxin M, Polymyxin E (Colistin) und Colistimethat. Die Wahl des spezifischen Polymyxin ist in das Belieben des Fachmannes gestellt und richtet sich u.a. nach der Indikation, dem Zustand des zu behandelnden Patienten, dem Verabreichungsweg und den toxikologischen Parametern.

Aminoglykoside bzw. Aminoglykosid-Antibiotika, zählen zur Gruppe der Oligosaccharid-Antibiotika, mit Kombinationen aus Aminozucker- und Cyclohexan-Bausteinen. Sie bilden eine große Gruppe von ca. 200 wasserlöslichen Antibiotika. Wichtige Vertreter sind Amikacin, Apramycin, Geneticin (G418), Gentamycin, Kanamycin, Netilmicin, Neomycin, Paromomycin, Spectinomycin, Streptomycin und Tobramycin. Die Aminoglykoside wirken stark bakterizid durch Hemmung der Proteinbiosynthese auf proliferierende und ruhende Erreger, indem sie an die 30 S-Untereinheit der Ribosomen ankoppeln und Ablesefehler der mRNA verursachen. Dadurch werden fehlerhafte Proteine gebildet, die vielfach nicht ihrer Funktion nachgehen können. In der Konsequenz werden z.B. defekte Proteine in die Zellmembran des Bakteriums eingebaut, was zur Lyse des Erregers führt.

Die Erfinder haben festgestellt, dass die Kombination der drei Wirkstoffe in einer synergistischen antibiotischen Wirkung resultiert. Dieser Synergismus war vor allem deshalb überraschend, da die drei kombinierten Substanzen unterschiedliche Wirkmechanismen ausüben.

Der Fachmann hätte aufgrund der unterschiedlichen Wirkmechanismen der drei Antibiotika chemische Interferenzen und ggf. eine Wirkungsneutralisierung erwartet. So konnten die Erfinder in parallelen Experimenten feststellen, dass bspw. die Kombination von Azthreonam-Lysin und Colistin weder synergistische noch additive Effekte zeigt. Der beobachtete Synergismus war deshalb besonders überraschend.

Ferner hätte der Fachmann eine Erhöhung der Nebenwirkungen vermutet. Überraschenderweise zeigte sich aber, dass die erfindungsgemäße pharmazeutische Zusammensetzung von Patienten sehr gut toleriert wurde. Auch aufgrund der insbesondere für Polymyxine und verschiedene Aminoglykoside im Stand der Technik beschriebenen Toxizitäten war dies nicht zu erwarten gewesen.

Als besonders vorteilhaft hat sich ferner herausgestellt, dass im Vergleich zur derzeitigen Therapie mit der erfindungsgemäßen pharmazeutischen Zusammensetzung die bakterizide Aktivität gegen eine Vielzahl von bakteriellen Spezies um ein Mehrfaches erhöht wird. Hinzu kommt, dass die erfindungsgemäße pharmazeutische Zusammensetzung die Entwicklung von Antibiotikaresistenzen aufgrund der neuen Kombination von Wirkstoffen.

Schließlich ist die erfindungsgemäße pharmazeutische Zusammensetzung kostengünstig herzustellen. Die drei Wirkstoffe sind im Markt verfügbar, pharmakologisch und toxikologisch umfassend getestet, was ein beschleunigtes Zulassungsverfahren begünstigt. Ferner ergibt sich Einsparungspotenzial dadurch, dass drei Wirkstoffe in einer einzelnen Zusammensetzung formuliert werden können.

Die erfindungsgemäße Zusammensetzung kann dabei derart ausgestaltet sein, dass sie als einzige arzneimittelwirksame Bestandteile ein Carbapenem, Polymyxin und Aminoglykosid enthält. Es versteht sich jedoch, dass weitere Wirkstoffe enthalten sein können, bspw. solche, die die erfindungsgemäße Wirksamkeit verstärken.

Die Wahl der jeweiligen Konzentrationen der Wirkstoffe wird von dem behandelnden Fachmann vorgenommen. Sie richtet sich u.a. nach der Indikation, dem Zustand des zu behandelnden Patienten, dem Verabreichungsweg und den toxikologischen Parametern.

Pharmazeutisch akzeptable Träger sind im Stand der Technik allgemein bekannt. Sie umfassen bspw. Bindemittel, Sprengmittel, Füllmittel, Gleitmittel sowie Puffer, Salze und sonstige zur Formulierung von Arzneimitteln geeignete Substanzen; vgl. Rowe et al. (2006), Handbook of Pharmaceutical Excipients, 5th Edition Pharmaceutical Press; oder Bauer et al. (1999), Lehrbuch der pharmazeutischen Technologie, 6. Auflage, Wissenschaftliche Verlagsgesellschaft Stuttgart mbH. Der Inhalt der vorliegenden Publikationen ist durch Inbezugnahme Bestandteil der vorliegenden Anmeldung. Besonders bevorzugt sind pharmazeutisch akzeptable Träger, die zur Formulierung von Antibiotika geeignet sind und/oder eine Verstärkung von Antibiotika-Wirkungen ermöglichen.

Nach einer bevorzugten Ausgestaltung ist die erfindungsgemäße pharmazeutische Zusammensetzung zur Behandlung einer Atemwegserkrankung ausgestaltet. Die Erfindung betrifft folglich auch die Verwendung einer Kombination von zumindest einem Carbapenem, Polymyxin und Aminoglykosid zur Behandlung einer Atemwegserkrankung bzw. ein Verfahren zur Herstellung eines Arzneimittels zur Behandlung einer Atemwegserkrankung, das folgende Schritte aufweist: (1) Bereitstellen einer Kombination von zumindest einem Carbapenem, Polymyxin und Aminoglykosid, und (2) Formulierung der Kombination in einen pharmazeutisch akzeptablen Träger.

Diese Maßnahme hat den Vorteil, dass drei bislang nur als Monopräparate eingesetzte und sich zur Behandlung von Atemwegserkrankungen bewährte Wirkstoffe nunmehr erstmalig in Kombination Verwendung finden, die aufgrund einer von den Erfindern erkannten synergistischen Wirkung und Nebenwirkungsarmut zur Behandlung einer Atemwegserkrankung besonders geeignet ist.

Dabei ist es erfindungsgemäß bevorzugt, wenn es sich bei dem Carbapenem um Meropenem oder Imipenem, und/oder bei dem Polymyxin um Colistin oder Colistimethat, und/oder bei dem Aminoglykosid um Tobramycin oder Gentamycin handelt.

Meropenem bzw. 3-[5-(Dimethylcarbamoyl)pyrrolidium-2-yl]sulfanyl-6-(1-hydroxyethyl)-4-methyl-7-oxo-1-azabicyclo[3.2.0]hept-2-en-2-carboxylsäure (CAS-Nummer 119478-56-7) ist ein Breitbandantibiotikum, das gegen eine Vielzahl von grampositiven und gramnegativen sowie anaeroben Bakterien wirksam ist. Es inhibiert die Synthese der bakteriellen Zellwand.

Imipenem bzw. (5R,6S)-6-[(1R)-1-hydroxyethyl]-3-({2-[(iminomethyl)amino]ethyl}thio)-7-oxo-1-azabicyclo[3.2.0]hept-2-ene-2-carboxylsäure (CAS-Nummer 74431-23-5) ist ein weiteres Antibiotikum mit breitem Wirkungsspektrum im grampositiven, gramnegativen, aeroben und anaeroben Bereich. Es wirkt durch Hemmung der Bakterienzellwandsynthese bakterizid.

Colistin bzw. N-(4-Amino-1-(1-(-4-amino-1-oxo-1-(3,12,23-tris(2-aminoethyl)-20-(1 -hydroxyethyl)-6,9-diisobutyl-2,5,8,11,14,19,22-heptaoxo-1,4,7,10,13,18-hexaazacyclotricosan-15-ylamino)butan-2-ylamino)-3-hydroxybutan-2-ylamino)-1-oxobutan-2-yl)-*N*,5-dimethylheptanamid (CAS-Nummer: 1264-72-8) ist ein gegen gram-negative Bakterien wirksames Antibiotikum. Colistin wirkt über eine Schädigung der äußeren Bakterienmembran. Colistimethat wird als Natriumsalz aus Colistin durch Umsetzen mit Formaldehyd und Natriumhydrogensulfit hergestellt. Es liegt als weißes Pulver vor, das in Wasser sehr leicht löslich ist.

Tobramycin bzw. (2*S*,3*R*,4*S*,5*S*,6*R*)-4-Amino-2-{[(1*S*,2*S*,3*R*,4*S*,6*R*]-4,6-diamino-3-{[(2*R*,3*R*,5*S*,6*R*)-3-amino-6-(aminomethyl)-4-hydroxyoxan-2-yl]oxy}-2-hydroxycyclohexyl]oxy}-6-(hydroxymethyl)oxan-3,5-diol (CAS-Nummer: 32986-56-4) ist ein gegen eine Vielzahl von Bakterien, vorzugsweise gramnegativen Bakterien, wirksames Antibiotikum. Tobramycin bindet an das bakterielle 30S- und 50S-Ribosom und verhindert so die Formierung des 70S-Komplexes. Im Ergebnis kann die mRNA nicht in ein Protein translatiert werden, wodurch es zum Zelltod kommt.

Gentamycin bzw. (3*R*,4*R*,5*R*)-2-{[(1*S*,2*S*,3*R*,4*S*,6*R*)-4,6-diamino-3-{[(2*R*,3*R*,6*S*)-3-amino-6-[(1*R*)-1-(methylamino)ethyl]oxan-2-yl]oxy}-2-hydroxycyclohexyl]oxy}-5-methyl-4-(methylamino)oxane-3,5-diol (CAS-Nummer 1403-66-3) ist ein Gemisch strukturell sehr ähnlicher Aminoglycosidverbindungen, den Gentamicinen. Es behindert das Ablesen der mRNA an den Ribosomen, durch Bindung an die 30S-Untereinheit. So wird die Proteinsynthese der Bakterien gebremst. Es wirkt vor allem gegen gramnegative Bakterien.

Wie die Erfinder feststellen konnten, lässt sich durch diese Auswahl eine noch gezieltere Behandlung einer Atemwegserkrankung realisieren.

Die erfindungsgemäße pharmazeutische Zusammensetzung ist für eine Applikation im Wege der Inhalation ausgestaltet.

Diese Maßnahme hat den Vorteil, dass die Wirkstoffe direkt an ihren Wirkort, nämlich in die Atemwege, gelangen und Nebenwirkungen sowie geringere Wirkstoffkonzentrationen in den Atemwegen, die bei systemischer Verabreichung auftreten würden, vermieden werden. Die Wirkung der erfindungsgemäßen Zusammensetzung wird dadurch optimiert.

Nach einer weiteren bevorzugten Ausgestaltung der erfindungsgemäßen pharmazeutischen Zusammensetzung weist der pharmazeutisch akzeptable Träger eine wässrige Chloridsalzlösung auf, die vorzugsweise hyperton ist.

Diese Maßnahme hat den unerwarteten Vorteil, die Wirksamkeit der erfindungsgemäßen Zusammensetzung auf synergistische Art und Weise zu erhöhen. Zu der verbesserten bakteriziden Wirkung der drei Antibiotika tritt eine sekretolytische Wirkung hinzu. Die vorzugsweise hypertone wässrige Chloridsalzlösung löst durch Wassereinstrom aus dem Lungengewebe die viskösen Mukusplaques und fördert damit die Selbstreinigung der Lunge. Gleichzeitig erleichtert sie die Wirkung der drei antibiotischen Wirkstoffe, was zu einer größeren Reduktion der bakteriellen Besiedelung der Atemwege führt. Dies resultiert in einer erheblichen Verbesserung der Lungenfunktion. Als besonders vorteilhaft hat sich dabei herausgestellt, dass die drei Wirkstoffe auch in hohen Konzentrationen von Chloridsalz löslich sind und ihre Wirksamkeit behalten.

Erfindungsgemäß wird unter einer hypertonen Chloridsalzlösung eine solche Lösung verstanden, die eine Chloridsalzkonzentration von > 0,9 % aufweist.

Nach einer bevorzugten Weiterbildung der erfindungsgemäßen pharmazeutischen Zusammensetzung ist die wässrige Chloridsalzlösung eine 6 %ige Chloridsalzlösung.

Diese Maßnahme hat den Vorteil, dass eine besonders gute sekretolytische Wirkung erzielt wird. Dabei war es überraschend, dass die Wirksamkeiten der drei Antibiotika durch die hohe Salzkonzentration nicht negativ beeinflusst werden.

Nach einer bevorzugten Weiterentwicklung der erfindungsgemäßen pharmazeutischen Zusammensetzung ist das Chloridsalz ausgewählt aus der Gruppe bestehend aus NaCl, MgCl und CaCl oder Mischungen hieraus.

Diese Maßnahme hat den Vorteil, dass ein solches Chloridsalz zum Einsatz kommt, welches nach Erkenntnissen der Erfinder zu besonders guten Ergebnissen führt.

Ferner ist es erfindungsgemäß bevorzugt, wenn die Atemwegserkrankung eine bakterielle Atemwegserkrankung ist.

Diese Maßnahme hat den Vorteil, dass die erfindungsgemäße pharmazeutische Zusammensetzung bei einem besonders problematischen Krankheitsbild zum Einsatz kommt und wirksam Abhilfe schafft.

Dabei ist es bevorzugt, wenn die bakterielle Atemwegserkrankung Bakterien involviert, die ausgewählt sind aus der Gruppe bestehend aus *Pseudomonas aeruginosa, Haemophilus influenzae* (HI) b, nicht-typisierbarer HI (NTHI), *Moraxella catarrhalis, Streptococcus pneumonia, Staphylococcus aureus, Burkholderia cepacia, Acinetobacter baumanii*, strikt-anaerobe Bakterien, einschließlich *Prevotella intermedia.*

Wie die Erfinder feststellen konnten, zeigt die erfindungsgemäße pharmazeutische Zusammensetzung bei den vorstehend genannten Bakterien, die sehr häufig in Zusammenhang mit Atemwegserkrankungen auftreten, positive Wirkung. Im Gegensatz zu den derzeit durchgeführten Therapien, die im Wesentlichen auf die Behandlung von *Pseudomonas aeruginosa* ausgerichtet sind, ist die erfindungsgemäße pharmazeutische Zusammensetzung durch ein deutlich breiteres Wirkspektrum gekennzeichnet und ermöglicht folglich die Behandlung auch solcher Atemwegserkrankungen, die mit der herkömmlichen Therapie nicht behandelbar sind.

Dabei ist es erfindungsgemäß bevorzugt, wenn die Atemwegserkrankung ausgewählt ist aus der Gruppe bestehend aus: chronisch obstruktiver Bronchitis (COPD), cystischer Fibrose (CF) und Ventilator-assoziierte Pneumonie (VAP).

Diese Maßnahme hat den Vorteil, dass eine pharmazeutische Zusammensetzung bereitgestellt wird, welche bei einigen der relevantesten Atemwegserkrankungen nutzbringend eingesetzt werden kann. So konnten die Erfinder insbesondere bei CF-Patienten, die erfindungsgemäß behandelt wurden, eine signifikante Reduzierung der Anzahl verschiedenster pathogener Bakterien und eine Erhöhung der ausgehusteten Sputummengen feststellen.

Die für die erfindungsgemäße pharmazeutische Zusammensetzung beschriebenen Merkmale, Eigenschaften und Vorteile gelten für die erfindungsgemäße Verwendung und das erfindungsgemäße Verfahren entsprechend.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Die Erfindung wird nun anhand von Ausführungsbeispielen näher beschrieben, aus denen sich weitere Merkmale, Eigenschaften und Vorteile ergeben. Die Ausführungsbeispiele sind rein illustrativ und schränken die Reichweite der Erfindung nicht ein.

Es wird Bezug genommen auf die beigefügten Figuren, in denen Folgendes dargestellt ist:

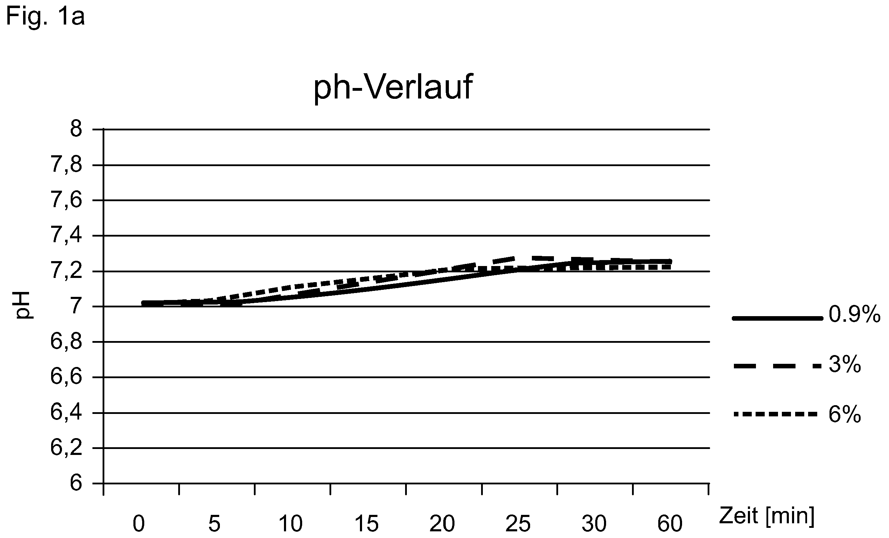

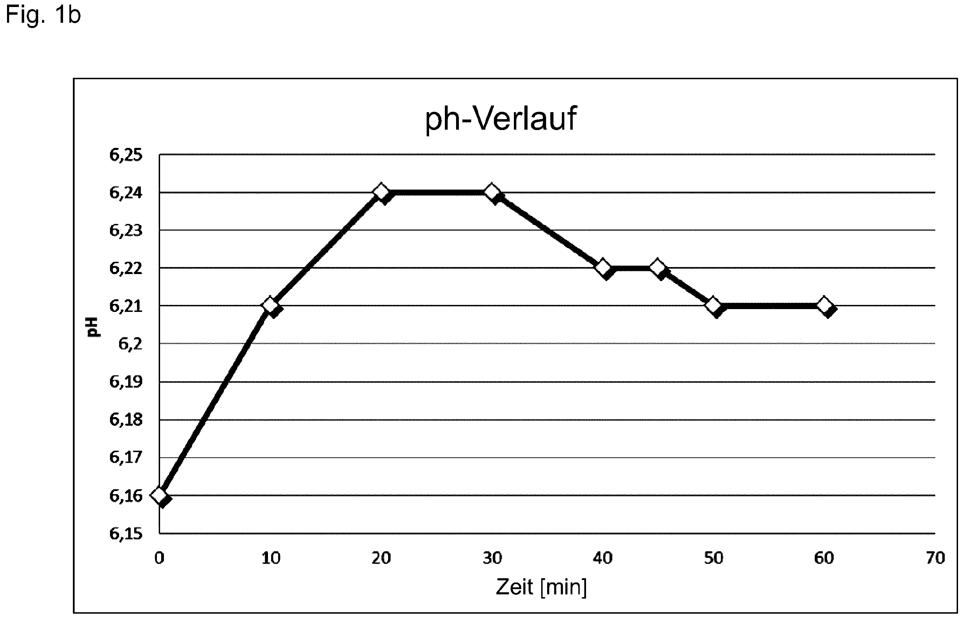

- Fig. 1: demonstriert anhand des pH-Wertes die Stabilität der erfindungsgemäßen pharmazeutischen Zusammensetzung bei verschiedenen Konzentrationen von Kochsalz, beispielhaft veranschaulicht an Kombinationen aus (a) Meropenem, Colistin und Tobramycin, und (b) Imipenem, Colistin und Gentamycin;

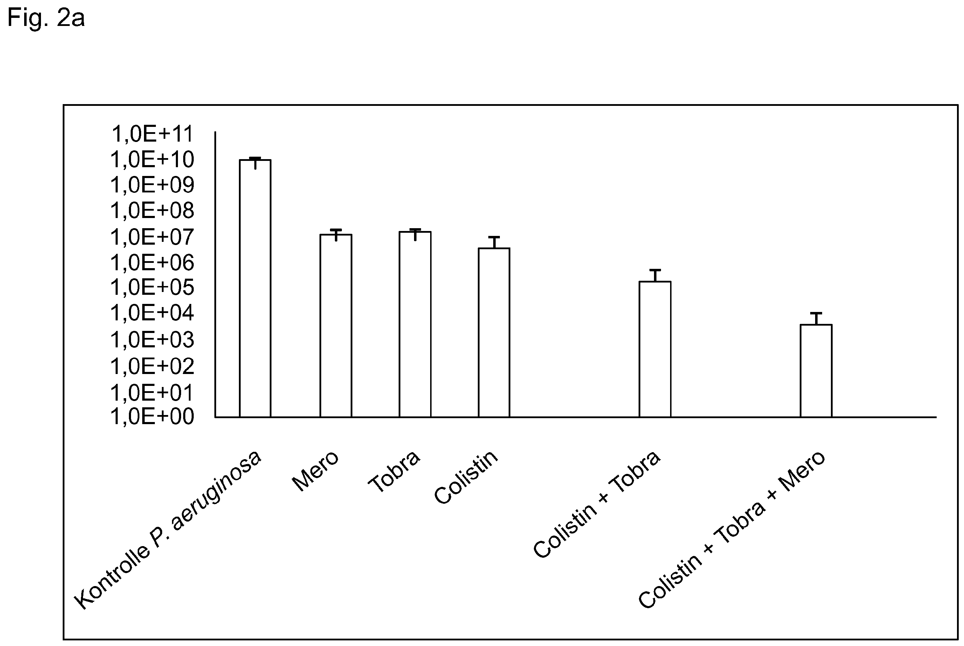

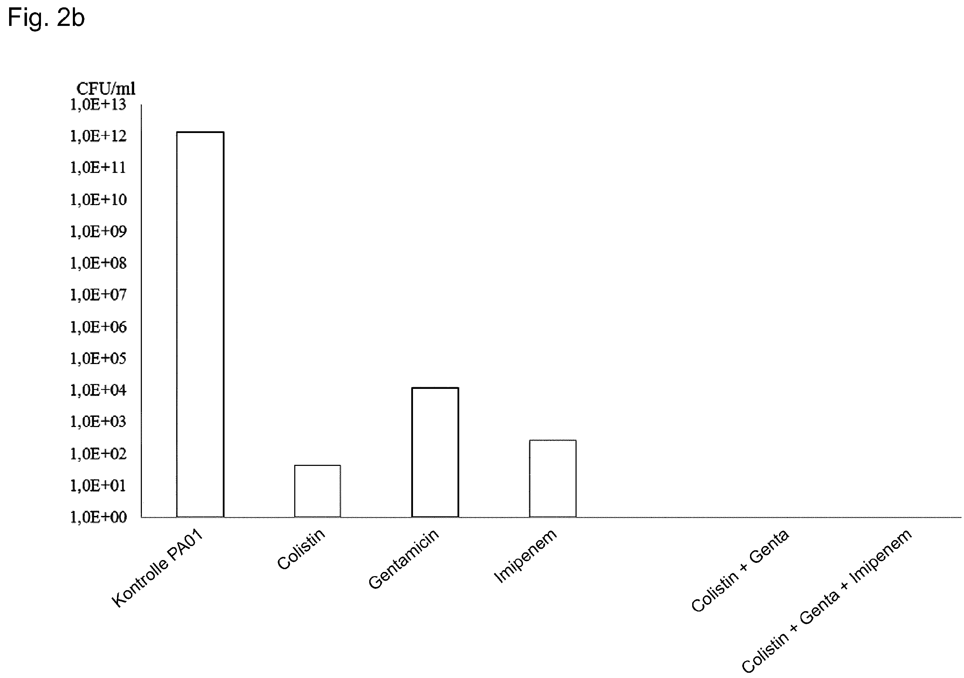

- Fig. 2: demonstriert die synergistische bakterizide Aktivität der in der erfindungsgemäßen pharmazeutischen Zusammensetzung kombinierten drei Antibiotika im Vergleich zur Verabreichung der einzelnen Antibiotika in Bezug auf das pathogene Bakterium *Pseudomonas aeruginosa,* beispielhaft veranschaulicht an Kombinationen aus (a) Meropenem, Colistin und Tobramycin, und (b) Imipenem, Colistin und Gentamycin;

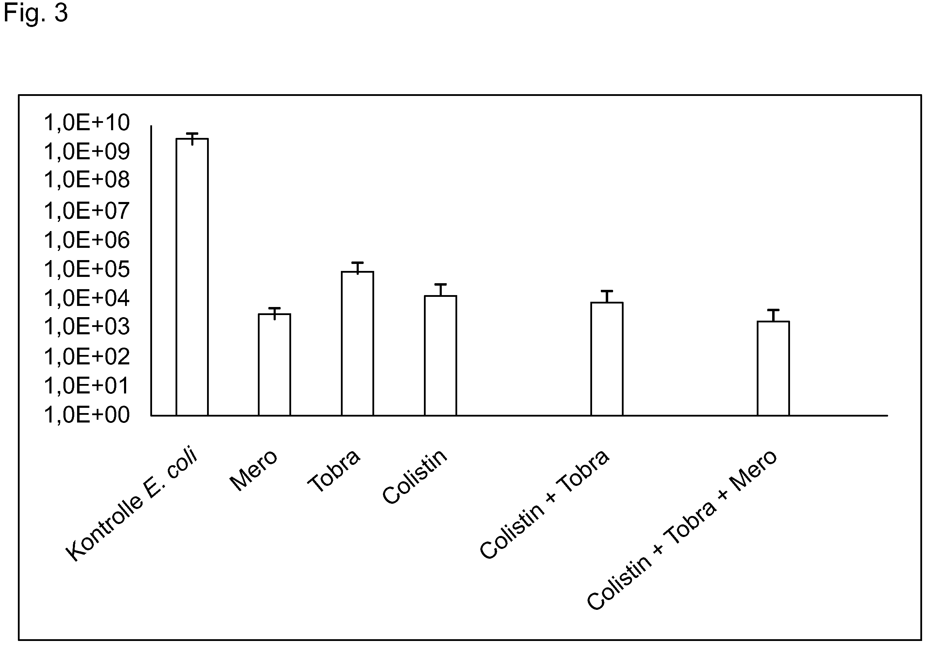

- Fig. 3: demonstriert die bakterizide Aktivität der in der erfindungsgemäßen pharmazeutischen Zusammensetzung kombinierten drei Antibiotika im Vergleich zur Verabreichung der einzelnen Antibiotika in Bezug auf das pathogene Bakterium *Escherichia coli,* beispielhaft veranschaulicht an der Kombination aus Meropenem, Colistin und Tobramycin.

### Ausführungsbeispiele

### 1. Chemische Stabilität der erfindungsgemäßen Kombination der drei Antibiotika bei verschiedenen Salzkonzentrationen

Die drei Antibiotika Meropenem, Colistin und Tobramycin wurden bei Konzentrationen von 60 mg/ml, 20 mg/ml und 75 mg/ml in 0,9 %iger, 3 %iger und 6 %iger wässriger Kochsalzlösung gelöst und die Stabilität der Lösungen über einen Zeitraum von 60 Minuten untersucht. Als Maß für die Stabilität diente dabei der pH-Wert. Weiterhin wurden die drei Antibiotika Imipenem, Colistin und Gentamycin bei Konzentrationen von 100 mg/ml, 20 mg/ml und 30 mg/ml in 0,9 %iger, wässriger Kochsalzlösung gelöst und die Stabilität der Lösungen über einen Zeitraum von 60 Minuten untersucht. Ferner wurde visuell untersucht, ob es zu Inkompatibilitäten wie Präzipitationen oder Verfärbungen kam.

Die Ergebnisse sind in den Figs. 1 a und 1 b dargestellt.

Dabei zeigte sich, dass alle drei Lösungen der drei Antibiotika Meropenem, Colistin und Tobramycin äußerst stabil waren. Es kam über die Zeit lediglich zu einer vernachlässigbaren minimalen Erhöhung des pH-Wertes, die in allen Fällen praktisch identisch auftrat. Ferner konnten keine Anzeichen für eine physikalische Inkompatibilität festgestellt werden. Es kam weder zu Präzipitationen noch zu Verfärbungen. Desgleichen war auch die Lösung von Imipenem, Colistin und Gentamycin in 0,9% Kochsalz über 60 min stabil. Es kam auch hier weder zu Präzipitationen noch zu Verfärbungen.

### 2. Synergistische bakterizide Aktivität der erfindungsgemäßen Kombination der drei Antibiotika in Bezug auf einen Stamm von Pseudomonas aeruginosa

Im nächsten Schritt wurde untersucht, ob sich die bakterizide Wirkung der einzelnen Antibiotika in Bezug auf einen Stamm von *Pseudomonas aeruginosa* bei pH 7 in einer 0,9 %igen Kochsalzlösung verändert, wenn die Wirkstoffe in Kombinationen eingesetzt werden. Dazu wurden die verschiedenen antibiotischen Wirkstoffe in Konzentrationen von 60 mg/ml Meropenem, 20 mg/ml Colistin und 75 mg/ml Tobramycin eingesetzt. Weiterhin wurden die drei Antibiotika Imipenem, Colistin und Gentamycin bei Konzentrationen von 100 mg/ml, 20 mg/ml und 30 mg/ml in 0,9 %iger, wässriger Kochsalzlösung eingesetzt.

Der Versuchsaufbau war wie folgt: Die Bakterien wurden auf Agar-Kulturplatten ausplattiert und mit den Antibiotikalösungen für 24 Stunden bei 37°C inkubiert. Anschließend wurden die Zonen der Inhibition des bakteriellen Wachstums innerhalb des Bakterienrasens gemessen.

Die Ergebnisse sind in den Figs. 2a und 2b dargestellt.

Dabei zeigte sich, dass durch die Kombination von Colistin, Tobramycin (Tobra) und Meropenem (Mero) gegenüber der Verabreichung der Einzelwirkstoffe eine signifikante Verbesserung der Wirksamkeit gegenüber *Pseudomonas aeruginosa* erreicht wird. Die Verabreichung der einzelnen Antibiotika reduzierte das ursprüngliche Inokulum des klinischen *Pseudomonas*-Isolates nur um ≤ 3 log₁₀ koloniebildende Einheiten (CFU)/ml, die Kombination von Colistin und Tobramycin bereits um 4 log₁₀ CFU/ml, und die erfindungsgemäße Kombination von Colistin, Tobramycin und Meropenem sogar um 6 log₁₀ CFU/ml.

Ähnliche Ergebnisse wurden mit der Kombination Imipenem, Colistin und Gentamycin (genta) erhalten. Die Verabreichung der einzelnen Antibiotika reduzierte das ursprüngliche Inokulum des Laborstammes PAO1 nur um circa ≤ 10 log₁₀koloniebildende Einheiten (CFU)/ml, die Kombination von Colistin und Gentamycin, sowie die Kombination von Imipenem, Colistin und Gentamycin jedoch vollständig.

Diese Wirkungssteigerung bei der erfindungsgemäßen Zusammensetzung ist auf einen Synergismus zurückzuführen. Dabei wird auf die Definition des "National Committee for Clinical Labaratory Standards" (NCCLS) aus dem Jahr 1999 verwiesen, das Synergismus als eine Reduzierung der bakteriellen CFU von ≥ 2 log₁₀ CFU/ml durch eine Kombination von verschiedenen Antibiotika im Vergleich zu der Reduzierung der bakteriellen CFU durch das aktivste einzelne Antibiotikum definiert; vgl. McCaughey et al. (2011), Synergistic Effects of a Fosfomycin:Tobramycin Combination on Cycstic Fibrosis Pathogens Grown. Aerobically and Anaerobically, Abstact form J. Cyct. Fibros. 10: Suppl. 1, 26, A100.

### 3. Bakterizide Aktivität der erfindungsgemäßen Kombination der drei Antibiotika in Bezug auf einen Stamm von Escherichia coli

Im nächsten Schritt wurde die Wirkung der erfindungsgemäßen Kombination der drei Antibiotika, beispielhaft demonstriert an Meropenem, Colistin und Tobramycin, in 0,9 %iger Kochsalzlösung bei einem pH 7 in Bezug auf einen Stamm von *Escherichia coli* untersucht. Der Versuchsaufbau entsprach dabei dem unter Ausführungsbeispiel 2 angegebenen, wobei der Stamm von *Pseudomonas aeruginosa* gegen einen Stamm von *Escherichia coli* ausgetauscht wurde.

Das Ergebnis ist in Fig. 3 dargestellt.

Dabei zeigte sich, dass die bei einer isolierten Verabreichung von Tobramycin bzw. Colistin beobachteten Wirkungen durch die erfindungsgemäße Kombination deutlich erhöht werden konnten. Die einzelnen Antibiotika reduzierten das ursprüngliche Inokulum um ≤ 6 log₁₀CFU/ml, wohingegen die erfindungsgemäße Kombination der drei Antibiotika in einer Reduktion von 6 log₁₀ CFU/ml resultierte.

### 4. Bakterizide Aktivität der erfindungsgemäßen Zusammensetzung bei verschiedenen Salzkonzentrationen

Als Nächstes wurde untersucht, welchen Einfluss die Salzkonzentration auf die bakterizide Aktivität der erfindungsgemäßen Kombination der drei Antibiotika, beispielhaft demonstriert an Meropenem, Colistin und Tobramycin, in Bezug auf vier klinische Isolate von *Pseudomonas aeruginosa* (1 bis 4) ausübt, die aus Sputumproben von CF-Patienten isoliert wurden. Hierzu wurden die Bakterien auf Agar-Kulturplatten ausplattiert und mit den unter Ziffern 1 und 2 beschriebenen Antibiotikalösungen bei verschiedenen in Wasser vorliegenden Salzkonzentrationen für 24 Stunden bei 37°C inkubiert. Anschließend wurden die Zonen der Inhibition des bakteriellen Wachstums innerhalb des Bakterienrasens gemessen.

Das Ergebnis ist in der nachstehenden Tabelle 1 dargestellt.

**Tabelle 1: Einfluss des Puffers mit zunehmenden Salzkonzentrationen auf die Potenz der erfindungsgemäßen Zusammensetzung in vitro.**

| Salzkonzentration | Zone der Inhibition des bakteriellen Wachstums (mm Durchmesser) Klinische Stämme von *Pseudomonas aeruginosa* | | | |
|---|---|---|---|---|
| | 1 | 2 | 3 | 4 |
| 0,9 % | 40 | 37 | 27 | 36 |
| 3 % | 42 | 37 | 29 | 36 |
| 6 % | 40 | 39 | 28 | 36 |

Dabei konnte im untersuchten Bereich kein signifikanter Einfluss der Salzkonzentration auf die Wirksamkeit der erfindungsgemäßen Zusammensetzung festgestellt werden.

### 5. Klinische Potenz der erfindungsgemäßen Kombination der drei Antibiotika in 6 %iger hypertoner Kochsalzlösung

In einer klinischen Studie wurde die Verträglichkeit und Wirksamkeit der erfindungsgemäßen Kombination von Meropenem, Colistin und Tobramycin in 6 %iger hypertoner Kochsalzlösung untersucht. Dazu wurden drei CF-Patienten [ein weiblicher und zwei männliche Patienten; durchschnittliches Alter 28 Jahre (18 bis 42 Jahre); mittlere 1-Sekunde-Kapazität (FEV₁: 53,2 %, vorhergesagt)] behandelt. Die erfindungsgemäße Zusammensetzung mit 60 mg/ml Meropenem, 20 mg/ml Colistin und 75 mg/ml Tobramycin wurde per einmaliger Inhalation unter Verwendung eines PARI eFlow oder eines Pari Master Verneblers verabreicht. Lungenfunktionstests wurde vor und eine Stunde nach der Inhalation durchgeführt. Ferner wurden vor und unmittelbar nach der Inhalation die CFU von *Pseudomonas aeruginosa, Staphylococcus aureus* und der gesamten Anaerobier im Sputum der Patienten bestimmt.

Dabei zeigte sich, dass die Inhalation der Lösung von den Patienten sehr gut toleriert wurde. Während der Studie zeigten sich, außer Husten während der Inhalation und einer reversiblen bronchialen Obstruktion, die sich innerhalb einer Stunde nach der Inhalation spontan auflöste, keine Nebenwirkungen. Der salzige Geschmack der Lösung wurde nicht als störend empfunden. Die Inhalationsbehandlung resultierte in einer erhöhten Sekretolyse, was sich visuell durch erhöhte Mengen von ausgehusteten Sputummengen beobachten ließ. Ferner wurde eine signifikante Reduzierung der Bakterienzahl (CFU) von *Pseudomonas aeruginosa* und *Staphylococcus aureus* sowie der Gesamtanaerobier festgestellt, was sich aus der Tabelle 2 ergibt.

**Tabelle 2: Klinische Potenz der erfindungsgemäßen Kombination der drei Antibiotika in 6 %iger hypertoner Kochsalzlösung - Erste bakteriologische Untersuchung im Menschen**

| Bakterien | Inhalation der Wirkstofflösung | | |
|---|---|---|---|
| | vor | nach | Signifikanz |
| | (Mittel log₁₀ CFU/ml ± SD) | | |
| *P. aeruginosa* | 7,12 ± 1,0 | 6,23 ± 1,1 | 0,003 |
| *S*. *aureus* | 5,66 ± 2,6 | 5,66 ± 2,6 | 0,37 |
| Gesamtanaearobier | 7,13 ± 0,04 | 4,72 ± 0,8 | 0,005 |

Wie den nachfolgenden Tabellen 3 und 4 zu entnehmen ist, führte die Verabreichung der erfindungsgemäßen Kombination der Antibiotika nicht zu einer Verschlechterung der Lungenfunktion. Im Gegenteil, bei dem maximalen expiratorischen Fluss bei 25, 50 und 75 % der forcierten Vitalkapazität (MEF₂₅₋₇₅) zeigte sich sogar eine signifikante Verbesserung.

**Tabelle 3: Klinische Potenz der erfindungsgemäßen Kombination der drei Antibiotika in 6 %iger hypertoner Kochsalzlösung - Erstuntersuchung im Menschen: Lungenfunktion FEV₁**

| Patient | Inhalation der Wirkstofflösung | | |
|---|---|---|---|
| | vor | nach | Signifikanz |
| | FEV₁ (% vorhergesagt ± SD) | | (p) |
| 1 | 47,9 | 44,0 | |
| 2 | 41,2 | 40,3 | |
| 3 | 70,4 | 73,6 | |
| Mittel | 53,2 ± 15 | 52,6 ± 18 | 0,41 |

**Tabelle 4: Klinische Potenz der erfindungsgemäßen Kombination der drei Antibiotika in 6 %iger hypertoner Kochsalzlösung - Erste Studie im Menschen: Lungenfunktion (MEF₂₅₋₇₅)**

| Patient | Inhalation der Wirkstofflösung | | |
|---|---|---|---|
| | vor | nach | Signifikanz |
| | MEF₂₅₋₇₅ (% | vorhergesagt ± SD) | (p) |
| 1 | 16,4 | 17,4 | |
| 2 | 12,0 | 11,5 | |
| 3 | 43,1 | 52,4 | |
| Mittel | 23,8 ± 17 | 27,1 ± 22 | 0,20 |

### 6. Fazit

Die Erfinder konnten eindrucksvoll anhand von Untersuchungen *in vitro* und *in vivo* demonstrieren, dass durch die Kombination von einem Carbapenem, Polymyxin und Aminoglykosid aufgrund einer synergistischen Wirkung effektiv Atemwegserkrankungen behandelt werden können. Die pharmazeutische Zusammensetzung kann in 6 %iger Salzlösung per Inhalation verabreicht werden, wodurch zusätzliche sekretolytische Wirkungen erzielt werden. Dabei sind in den Antibiotikaklassen der Carbapeneme und der Aminoglykoside mindestens 2 Vertreter mit ähnlich gutem Wirkungsgrad erfolgreich untersucht worden. Die erfindungsgemäße pharmazeutische Zusammensetzung zeichnet sich durch gute Verträglichkeit und geringe Nebenwirkungen aus.

## Patentansprüche

1. Pharmazeutische Zusammensetzung aufweisend eine Kombination von zumindest einem Carbapenem, Polymyxin und Aminoglykosid sowie einen pharmazeutisch akzeptablen Träger, **dadurch gekennzeichnet, dass** die pharmazeutische Zusammensetzung für eine Applikation im Wege der Inhalation ausgestaltet ist.

2. Pharmazeutische Zusammensetzung nach Anspruch 1, **dadurch gekennzeichnet, dass** das Carbapenem Meropenem oder Imipenem ist.

3. Pharmazeutische Zusammensetzung nach Anspruch 1 oder 2, **dadurch gekennzeichnet, dass** das Polymyxin Colistin oder Colistimethat ist.

4. Pharmazeutische Zusammensetzung nach einem der Ansprüche 1 bis 3, **dadurch gekennzeichnet, dass** das Aminoglykosid Tobramycin oder Gentamycin ist.

5. Pharmazeutische Zusammensetzung nach einem der Ansprüche 1 bis 4 zur Verwendung zur Behandlung einer Atemwegserkrankung.

6. Pharmazeutische Zusammensetzung nach einem der vorherigen Ansprüche, **dadurch gekennzeichnet, dass** der pharmazeutisch akzeptable Träger eine wässrige Chloridsalzlösung aufweist.

7. Pharmazeutische Zusammensetzung nach Anspruch 6, **dadurch gekennzeichnet, dass** die Chloridsalzlösung hyperton ist.

8. Pharmazeutische Zusammensetzung nach Anspruch 7, **dadurch gekennzeichnet, dass** die Chloridsalzlösung eine ca. 6 %ige Chloridsalzlösung ist.

9. Pharmazeutische Zusammensetzung nach einem der Ansprüche 6 bis 8, **dadurch gekennzeichnet, dass** das Chloridsalz ausgewählt ist aus der Gruppe bestehend aus: NaCl, MgCl und CaCl oder Mischungen hieraus.

10. Pharmazeutische Zusammensetzung nach einem der Ansprüche 5 bis 9, **dadurch gekennzeichnet, dass** die Atemwegserkrankung eine bakterielle Atemwegserkrankung ist.

11. Pharmazeutische Zusammensetzung nach Anspruch 10, **dadurch gekennzeichnet, dass** die bakterielle Atemwegserkrankung Bakterien involviert, die ausgewählt sind aus der Gruppe bestehend aus: *Pseudomonas aeruginosa, Haemophilus influenzae* (HI) b, nicht-typisierbarer HI (NTHI), *Moraxella catarrhalis, Streptococcus pneumonia, Staphylococcus aureus, Burkholderia cepacia, Acinetobacter baumanii*, strikt-anaerobe Bakterien, einschließlich *Prevotella intermedia.*

12. Pharmazeutische Zusammensetzung nach einem der Ansprüche 5 bis 11, **dadurch gekennzeichnet, dass** die Atemwegserkrankung ausgewählt ist aus der Gruppe bestehend aus: Chronisch obstruktiver Bronchitis (COPD), cystischer Fibrose (CF) und Ventilator-assoziierte Pneumonie (VAP).

## Claims

1. Pharmaceutical composition comprising a combination of at least one carbapenem, polymyxin, and aminoglycoside and a pharmaceutically acceptable carrier, **characterized in that** the pharmaceutical composition is configured for an application by way of inhalation.

2. Pharmaceutical composition of claim 1, **characterized in that** the carbapenem is meropenem or imipenem.

3. Pharmaceutical composition of claim 1 or 2, **characterized in that** the Polymyxin is colistin or colistimethat.

4. Pharmaceutical composition of any of claims 1 to 3, **characterized in that** the aminoglycoside is tobramycin or gentamycin.

5. Pharmaceutical composition of any of claims 1 to 4 for a use in the treatment of a respiratory disease.

6. Pharmaceutical composition of any of the previous claims, **characterized in that** the pharmaceutically acceptable carrier comprises an aqueous chlorite salt solution.

7. Pharmaceutical composition of claim 6, **characterized in that** the chlorite salt solution is hypertonic.

8. Pharmaceutical composition of claim 7, **characterized in that** the chlorite salt solution is an approximately 6% chlorite salt solution.

9. Pharmaceutical composition of any of the claims 6 to 8, **characterized in that** the chlorite salt is selected from the group consisting of: NaCl, MgCl, and CaCl or mixtures thereof.

10. Pharmaceutical composition of any of claims 5 to 9, **characterized in that** the respiratory disease is a bacterial respiratory disease.

11. Pharmaceutical composition of claim 10, **characterized in that** the bacterial respiratory disease involves bacteria which are selected from the group consisting of: *Pseudomonas aeruginosa, Haemophilus influenzae* (HI) b, HI non-typable (NTHI), *Moraxella catarrhalis, Streptococcus pneumonia, Staphylococcus aureus, Burkholderia cepacia, Acinetobacter baumanii,* strictly unaerobic bacteria, including *Prevotella intermedia.*

12. Pharmaceutical composition of any of claims 5 to 11, **characterized in that** the respiratory disease is selected from the group consisting of: chronic obstructive pulmonary disease (COPD), cystic fibrosis (CF), and ventilator-associated pneumonia (VAP).

## Revendications

1. Composition pharmaceutique présentant une combinaison d'au moins un carbapénème, une polymyxine et un aminoglycoside ainsi qu'un véhicule pharmaceutiquement acceptable, **caractérisée en ce que** la composition pharmaceutique est conçue pour une application dans des voies d'inhalation.

2. Composition pharmaceutique selon la revendication 1, **caractérisée en ce que** le carbapénème est le méropénème ou l'imipénème.

3. Composition pharmaceutique selon la revendication 1 ou 2, **caractérisée en ce que** la polymyxine est la colistine ou le colistiméthate.

4. Composition pharmaceutique selon l'une quelconque des revendications 1 à 3, **caractérisée en ce que** l'aminoglycoside est la tobramycine ou la gentamycine.

5. Composition pharmaceutique selon l'une quelconque des revendications 1 à 4 pour son utilisation pour le traitement d'une maladie des voies respiratoires.

6. Composition pharmaceutique selon l'une quelconque des revendications précédentes, **caractérisée en ce que** le véhicule pharmaceutiquement acceptable est une solution aqueuse d'un sel de chlorure.

7. Composition pharmaceutique selon la revendication 6, **caractérisée en ce que** la solution de sel de chlorure est hypertonique.

8. Composition pharmaceutique selon la revendication 7, **caractérisée en ce que** la solution de sel de chlorure est une solution de sel de chlorure à environ 6 %.

9. Composition pharmaceutique selon l'une quelconque des revendications 6 à 8, **caractérisée en ce que** le sel de chlorure est choisi dans le groupe comprenant : le NaCl, le MgCl et le CaCI ou leurs mélanges.

10. Composition pharmaceutique selon l'une quelconque des revendications 5 à 9, **caractérisée en ce que** la maladie des voies respiratoires est une maladie bactérienne des voies respiratoires.

11. Composition pharmaceutique selon la revendication 10, **caractérisée en ce que** la maladie bactérienne des voies respiratoires implique des bactéries qui sont choisies dans le groupe comprenant : *Pseudomonas aeruginosa, Haemophilus influenza* (HI) b, HI non typable (NTHI), *Moraxella catarrhalis, Streptococcus pneumonia, Staphylococcus aureus, Burkholderia cepacia, Acinetobacter baumanii,* des bactéries strictement anaérobies comprenant *Prevotella intermedia.*

12. Composition pharmaceutique selon l'une quelconque des revendications 5 à 11, **caractérisée en ce que** la maladie des voies respiratoire est choisie dans le groupe comprenant : la broncho-pneumopathie chronique obstructive (BPCO), la fibrose kystique (FK) et la pneumopathie acquise sous ventilation (PAV).
